19 Europäisches Patentamt

European Patent Office

Office européen des brevets

11 Veröffentlichungsnummer: **0 282 778 B1**

12 # EUROPÄISCHE PATENTSCHRIFT

45 Veröffentlichungstag der Patentschrift: **13.05.92**

51 Int. Cl.5: **C07D 215/48**

21 Anmeldenummer: **88102684.3**

22 Anmeldetag: **24.02.88**

Die Akte enthält technische Angaben, die nach dem Eingang der Anmeldung eingereicht wurden und die nicht in dieser Patentschrift enthalten sind.

54 **Verfahren zur Herstellung von 7-Chlor-chinolin-8-carbonsäuren.**

30 Priorität: **03.03.87 DE 3706792**
**12.12.87 DE 3742267**

43 Veröffentlichungstag der Anmeldung:
**21.09.88 Patentblatt 88/38**

45 Bekanntmachung des Hinweises auf die Patenterteilung:
**13.05.92 Patentblatt 92/20**

84 Benannte Vertragsstaaten:
**BE CH DE FR GB LI NL**

56 Entgegenhaltungen:
**EP-A- 0 060 429**
**EP-A- 0 085 182**
**EP-A- 0 104 389**

73 Patentinhaber: **BASF Aktiengesellschaft**
**Carl-Bosch-Strasse 38**
**W-6700 Ludwigshafen(DE)**

72 Erfinder: **Hagen, Helmut, Dr.**
**Max-Slevogt-Strasse 17 e**
**W-6710 Frankenthal(DE)**
Erfinder: **Dupuis, Jaques Dr.**
**Bannwasserstrasse 37**
**W-6700 Ludwigshafen(DE)**
Erfinder: **Eilingsfeld, Heinz, Dr.**
**Pierstrasse 9a**
**W-6710 Frankenthal(DE)**

## Beschreibung

Die bekannte Herstellung von 7-Chlor-3-methylchinolin-8-carbonsäure verläuft über die Zwischenstufe der entsprechenden Brommethylverbindung. Dieses aus der EP-A-104 389 bekannte Verfahren hat den Nachteil, daß eine zusätzliche Reaktionsstufe und somit mehr Aufwand erforderlich ist und liefert das gewünschte Zielprodukt nur mit geringer Ausbeute.

Die Erfindung stellt ein Verfahren zur Herstellung von 7-Chlor-3-methylchinolin-8-carbonsäure durch Oxidation von 7-Chlor-3,8-dimethylchinolin bei erhöhter Temperatur zur Verfügung, das dadurch gekennzeichnet ist, daß man 7-Chlor-3,8-dimethylchinolin in Schwefelsäure mit Salpetersäure oder Stickstoffdioxid in Gegenwart katalytischer Mengen einer Vanadium- und/oder Kobaltverbindung bei Temperaturen von 120 bis 180°C in Gegenwart von Sauerstoff selektiv an der 8-Methylgruppe oxidiert.

Die verbrauchte Salpetersäure bzw. die Stickstoffoxide können mit molekularem Sauerstoff regeneriert werden.

Das Verfahren bedeutet eine erhebliche Verbesserung hinsichtlich des Aufwandes, der Ausbeute und der Reinheit des Zielproduktes.

Die Vanadium und/oder Kobaltverbindungen können einzeln oder als Mischung eingesetzt werden, vorzugsweise in Form ihrer in Schwefelsäure bzw. Gemischen von Schwefelsäure und Salpetersäure löslichen Verbindungen. Verbindungen in diesem Sinne sind insbesondere die Oxide und natürlich die Sulfate, ferner Nitrate und Acetate. Das Schwermetall wird in katalytischer Menge benötigt, also im allgemeinen weniger als 10, insbesondere weniger als 5 Gew.-%, bezogen auf das Reaktionsgemisch, in dem es wirken soll. Auch sehr geringe Mengen, z.B. weniger als 1 Gew.-%, sind durchaus wirksam.

Molekularer Sauerstoff wird zweckmäßig in Form von Luft, etwa durch kräftiges Einleiten in das System gebracht und bewirkt eine Erhöhung der Selektivität und eine Reaktionsbeschleunigung.

Durch eine besondere Ausgestaltung der Erfindung läßt sich die vorgestellte Herstellung hinsichtlich des Aufwandes und der Flexibilität verbessern: Man kann die Direktoxidation mit Salpetersäure oder mit Stickstoffdioxid unter Rückführung des NO$_x$-haltigen Reaktionsabgases vornehmen. Man führt zweckmäßig das Abgas im Kreis und bringt es dabei, wenn gewünscht, mit dem molekularen Sauerstoff, praktisch also mit Luft, in Verbindung, wodurch es regeneriert wird.

Die Umsetzung wird bei einer Temperatur von z.B. 120 bis 180, insbesondere 150 bis 170°C durchgeführt. Dabei liegt eine Lösung von 7-Chlor-3,8-dimethylchinolin in z.B. 50 bis 90 %iger - bevorzugt 70 bis 85 %iger Schwefelsäure vor und es wird ggf. mäßig konzentrierte oder konzentrierte Salpetersäure verwendet. Praktisch geht man z.B. so vor, daß man das 7-Chlor-3,8-dimethylchinolin in der den Katalysator enthaltenden Schwefelsäure bei 150 bis 160°C vorlegt und dann allmählich mit etwa 3 bis 6 Äquivalenten Salpetersäure - bezogen auf 1 mol 7-Chlor-3,8-dimethylchinolin - versetzt bzw. einfach gasförmiges Stickstoffdioxid einleitet. Der Sättigungsdruck kann atmosphärischer Druck oder ein geringfügig höherer Druck sein. Die Umsetzung verläuft innerhalb von 8 bis 15 Stunden praktisch vollständig.

Man verdünnt mit Wasser, bringt auf pH 0,5 bis 3 (wobei alle Chinolinverbindungen einschließlich eventueller Nebenprodukte ausfallen) und gewinnt die Carbonsäure durch Ausziehen mit Methanol, Isopropylalkohol o.ä. als Rückstand, der durch Umkristallisation wenn nötig gereinigt werden kann.

Die Umsetzung kann absatzweise z.B. im Rührkessel oder nach den üblichen Regeln der Verfahrenstechnik auch fortlaufend vorgenommen werden, wobei z.B. ein Rührkessel mit nachgeschaltetem Rohrreaktor, eine Rührkessel kaskade oder ein Rohrreaktor verwendbar sind; für die Umsetzung mit gasförmig zugeführten Reaktionsteilnehmern ist die Verwendung z.B. einer Blasensäule oder eines Gasstrahlreaktors von Vorteil. Einschlägig verwendbare Verfahrensanordnungen sind dem verfahrenstechnisch ausgebildeten Fachmann zugänglich.

Um ein besonders günstiges Ergebnis zu erzielen, geht man von möglichst reinem 7-Chlor-3,8-dimethylchinolin aus; mit gutem Erfolg kann man aber auch unmittelbar die Reaktionsgemische verwenden, die - in schwefelsaurer Lösung - eine entsprechende Methylchinolinverbindung enthalten, wie sie bei der Chinolinsynthese nach Skraup aus einem entsprechend substituierten Toluidin und (Meth)acrolein anfallen.

Beispiel 1

Herstellung von 7-Chlor-3-methylchinolin-8-carbonsäure

191,5 g (1 mol) 7-Chlor-3,8-dimethylchinolin werden in 2400 g 70 %iger Schwefelsäure gelöst, auf 140°C erwärmt und 2,5 g Vanadium(V)oxid und 1,3 g Cobalt(II)acetat zugegeben. 339 g 65 %ige Salpetersäure werden innerhalb von 13 bis 15 Stunden zugesetzt. Mit der Zugabe der Salpetersäure beginnt man auch, Luft einzuleiten. Der Luftstrom soll ausreichen, die entstehenden nitrosen Gase zu entfernen. Nach dem Ende der Umsetzung rührt man noch 1 bis 2 Stunden weiter, kühlt ab und verdünnt mit Wasser auf das Doppelte.

Ein pH-Wert von 0,5 wird mit konzentrierter Natronlauge eingestellt und der Niederschlag abfiltriert. Der feuchte Filterkuchen wird zweimal mit der gleichen Menge Methanol oder Isopropylalkohol aufgekocht und bei 40°C filtriert. Die erhaltene Säure (199 g $\widehat{=}$ 69 % d. Th.) hat eine Reinheit von 95 %, Wenn höhere Reinheit erfordert wird, kann man vor der Alkoholbehandlung aus verdünnter Natronlauge umfällen.

Beispiel 2

Man verfährt, wie in Beispiel 1 beschrieben, unter Verwendung von ausschließlich 2,5 g Vanadium(V)oxid und gewinnt 152 g (69 %) des Zielprodukts.

Beispiel 3

191,5 g (1 mol) 7-Chlor-3,8-dimethylchinolin werden in 1730 g 80 %iger Schwefelsäure gelöst, auf 150 °C erwärmt und 2,5 g Vanadium(V)oxid zugegeben. 418 g 65 %ige Salpetersäure werden innerhalb von 13 bis 15 Stunden zugesetzt. Mit der Zugabe der Salpetersäure beginnt man auch das $NO_X$-haltige Abgas umzupumpen, d. h. im Kreis zu führen. Nach dem Ende der Umsetzung kühlt man ab und verdünnt mit Wasser auf das Doppelte.
Ein pH-Wert von 0,5 wird mit konzentrierter Natronlauge eingestellt und der Niederschlag abfiltriert. Man erhält 183,3 g trockene Rohware die 83 Gew.-% (159 g) Chinolincarbonsäure enthält (79 % d. Th.).

Beispiel 4

Man verfährt, wie in Beispiel 3 beschrieben, die gleiche Menge Salpetersäure wird in 7 Std. zugegeben. Man gewinnt 188.4 g trockene Rohware mit 87 Gew.-% Chinolincarbonsäure (74 % d. Th.).

Beispiel 5

574,5 g (3 mol) 7-Chlor-3,8-dimethylchinolin werden in 1730 g 80 %iger Schwefelsäure gelöst und 7,5 g Vanadiumpentoxid zugegeben. 1254 g 65 %ige Salpetersäure werden innerhalb von 14 Std. zugesetzt. In dieser Zeit wird das $NO_X$-haltige Abgas umgepumpt. Nach der Fällung wie in Beispiel 1 werden 508,6 g Rohware erhalten. Der Gehalt an Chinolincarbonsäure beträgt 87 % (Ausbeute 62 % d. Th.).

Beispiel 6

574,5 g (3 mol) 7-Chlor-3,8-dimethylchinolin werden in 1780 g 85 %iger Schwefelsäure gelöst. 10 g Vanadiumpentoxid werden hinzugegeben. Bei 160°C wird die Apparatur mit $NO_2$ gesättigt bis zu einem Überdruck von 0,2 bar. Durch kontinuierliche Sauerstoffzufuhr wird ein Überdruck von 0,5 bar konstant gehalten; nach 14 h wird kein Sauerstoff mehr aufgenommen. Der Reaktor wird entspannt und der Inhalt wie unter Beispiel 1 beschrieben aufgearbeitet. Man erhält 558 g Chinolincarbonsäure mit einem Reingehalt von 89 % (Ausbeute 75 % d. Th.).

Beispiel 7

574,5 g (3 mol) 7-Chlor-3,8-dimethylchinolin werden in 1780 g 85 %iger Schwefelsäure gelöst. 10 g Vanadiumpentoxid werden hinzugegeben. Bei 160°C wird $NO_2$ in einer Menge von 200 Litern pro Stunde eingeleitet und das $NO_X$-haltige Abgas umgepumpt. Nach 7 Stunden ist die Umsetzung beendet. Das Reaktionsgemisch wird verdünnt mit Natronlauge, auf pH 0,5 gebracht und der Niederschlag abfiltriert und getrocknet. Man erhält 562 g der rohen Chinolincarbonsäure, die einen Gehalt von 87 % aufweist (Ausbeute 73 % d. Th.).

Beispiel 8

806 g VE-Wasser, 2208 g konz. Schwefelsäure und 566 g (4 mol) 3-Chlor-2-methylanilin werden vorgelegt; bei 40°C wird eine Lösung von 8 g Natriumiodid in 16 g Wasser hinzugegeben. Bei 120°C werden 336 g (4,8 mol) Methacrolein innerhalb von 2 Stunden zudosiert. Anschließend gibt man 100 mg eines handelsüblichen Schaumdämpfers zu und kreist 750 g Wasser aus, indem die Temperatur auf 160°C gebracht wird. Nach Zugabe von 15 g Vanadiumpentoxid leitet man gasförmiges $NO_2$ (200 1/h) ein. Das Reaktionsabgas wird umgepumpt, wobei man die Umwälzgeschwindigkeit innerhalb von 2 Stunden von 100 auf 1600 1/h erhöht. Nach 8 Stunden ist die Reaktion beendet. Man erhält nach der Fällung bei pH 0.5 727 g Rohprodukt mit einem Gehalt von 67 % (Ausbeute 54 % d` Th.).

**Patentansprüche**

1. Verfahren zur Herstellung von 7-Chlor-3-methylchinolin-8-carbonsäure durch Oxidation von 7-Chlor-3,8-dimethylchinolin bei erhöhter Temperatur, dadurch gekennzeichnet, daß man 7-Chlor-3,8-dimethylchinolin in Schwefelsäure mit Salpetersäure oder Stickstoffdioxid in Gegenwart katalytischer Mengen einer Vanadium-

und/oder Kobaltverbindung bei Temperaturen von 120 bis 180°C in Gegenwart von Sauerstoff selektiv an der 8-Methylgruppe oxidiert.

2. Verfahren nach Anspruch 1, dadurch gekennzeichnet, daß man die Umsetzung in Gegenwart bzw. unter Einleiten von Luft vornimmt.

3. Verfahren nach Anspruch 1, dadurch gekennzeichnet, daß man die Oxidation unter Rückführung der $NO_x$-haltigen Abgase durchführt.

4. Verfahren nach Anspruch 3, dadurch gekennzeichnet, daß man die Abgase mit Sauerstoff bzw. Luft behandelt.

5. Verfahren nach einem der Ansprüche 1 bis 4, dadurch gekennzeichnet, daß man das 7-Chlor-3,8-dimethylchinolin in Form des nach Skraup erhaltenen schwefelsauren Reaktionsgemischs verwendet.

**Claims**

1. A process for preparing 7-chloro-3-methylquinoline-8-carboxylic acid by oxidation of 7-chloro-3,8-dimethylquinoline at elevated temperature, which comprises oxidizing 7-chloro-3,8-dimethylquinoline selectively at the 8-methyl group with nitric acid or nitrogen dioxide at 120-180°C in sulfuric acid in the presence of oxygen and a catalytic amount of a vanadium or cobalt compound.

2. A process as claimed in claim 1, wherein the reaction is carried out in the presence of, or by passing in, air.

3. A process as claimed in claim 1, wherein the oxidation is carried out with recycling of the $NO_x$-containing offgases.

4. A process as claimed in claim 3, wherein the offgases are treated with oxygen or air.

5. A process as claimed in any of claims 1 to 4, wherein the 7-chloro-3,8-dimethylquinoline is used in the form of the sulfuric acid reaction mixture from a Skraup synthesis.

**Revendications**

1. Procédé de préparation de l'acide 7-chloro-3-méthylquinoléine-8-carboxylique par oxydation de la 7-chloro-3,8-diméthylquinoléine à haute température, caractérisé en ce que l'on oxyde la 7-chloro-3,8-diméthylquinoléine sélectivement sur le groupe 8-méthyle dans l'acide sulfurique par l'acide nitrique ou le peroxyde d'azote en présence de quantités catalytiques d'un composé du vanadium et/ou du cobalt, à des températures de 120 à 180 degrés C en présence d'oxygène.

2. Procédé selon la revendication 1, caractérisé en ce que l'on effectue la réaction en présence d'air ou sous injection d'air.

3. Procédé selon la revendication 1, caractérisé en ce que l'on effectue l'oxydation avec recyclage des gaz résiduaires contenant les oxydes d'azote.

4. Procédé selon la revendication 3, caractérisé en ce que l'on traite les gaz résiduaires par l'oxygène ou l'air.

5. Procédé selon l'une de revendications 1 à 4, caractérisé en ce que l'on met en oeuvre la 7-chloro-3,8-diméthylquinoléine à l'état du mélange de réaction contenant de l'acide sulfurique obtenu selon Skraup.